(19) Europäisches Patentamt
European Patent Office
Office européen des brevets

(11) Publication number: **0 539 587 A1**

# (12) EUROPEAN PATENT APPLICATION published in accordance with Art. 158(3) EPC

(21) Application number: **91909362.5**

(51) Int. Cl.[5]: **A61K 31/70**, //C07H13/08

(22) Date of filing: **16.05.91**

(86) International application number:
**PCT/JP91/00651**

(87) International publication number:
**WO 92/20345 (26.11.92 92/29)**

(43) Date of publication of application:
**05.05.93 Bulletin 93/18**

(84) Designated Contracting States:
**FR**

(71) Applicant: **TSUMURA & CO.**
**4-10, Nihonbashi 3-chome**
**Chuo-ku, Tokyo 103(JP)**

(72) Inventor: **HIGUCHI, Hirotaka**
**Tsumura & Co. 3586, Yoshiwara Ami-machi**
**Inashiki-gun Ibaraki 300-11(JP)**
Inventor: **OGATA, Takahiro**
**Tsumura & Co. 3586, Yoshiwara Ami-machi**
**Inashiki-gun Ibaraki 300-11(JP)**
Inventor: **MATSUMOTO, Hideki**
**Tsumra & Co. 3586, Yoshiwara Ami-machi**
**Inashiki-gun Ibaraki 300-11(JP)**
Inventor: **KATO, Akihisa**
**Tsumura & Co. 3586, Yoshiwara Ami-machi**
**Inashiki-gun Ibaraki 300-11(JP)**

(74) Representative: **Hirsch, Marc-Roger et al**
**Cabinet Hirsch 34 rue de Bassano**
**F-75008 Paris (FR)**

## (54) ANTIRETROVIRAL AGENT.

(57) An antiretroviral agent containing as the active ingredient a compound of general formula (I) or a pharmaceutically acceptable salt thereof, wherein $R_1$ and $R_2$ may be the same or different from each other and each represents hydrogen, sodium or potassium.

EP 0 539 587 A1

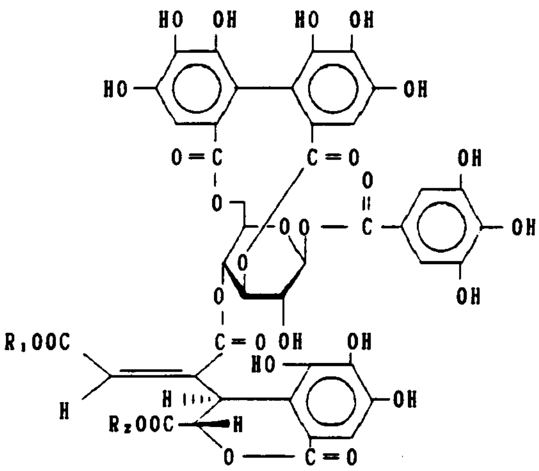

(I)

## TECHNICAL FIELD

The present invention relates to an anti-retroviral agent that is effective in the treatment of various types of viral diseases caused by retroviruses, and demonstrates high solubility in water.

## BACKGROUND ART

As more and more research is conducted pertaining to viruses, a growing-number of treatment methods for viral diseases are gradually being established.

Human immunodeficiency virus (HIV), which causes acquired immunodeficiency syndrome (AIDS), a recent problem of particular concern, is known to be a retrovirus.

Retroviruses are viruses that contain an RNA-dependent DNA synthesizing enzyme (to be referred to as reverse transcriptase) within viral particles, and reproduce in the manner described below.

(1) Following infection of the host cell, viral RNA is first transcribed into DNA by reverse transcriptase.

(2) This DNA is then incorporated into the chromosomes of the host cell followed by synthesis of mRNA by an RNA synthesizing enzyme of the host cell.

(3) Various viral proteins are then synthesized by this mRNA.

As such, there is a need for the development of a medication that possesses revolutionary therapeutic effects against human diseases caused by these retroviruses.

## DISCLOSURE OF THE INVENTION

As a result of conducting research relating to retrovirus growth inhibitory effects of ingredients contained in Phyllanthus Niruri L., a plant native to Taiwan and other countries, the inventors of the present invention discovered that retrovirus growth inhibitory effects are present in a compound represented by the following formula, thus leading to completion of the present invention.

More specifically, the present invention provides an anti-retroviral agent comprising, as an active ingredient, a compound represented by the following formula:

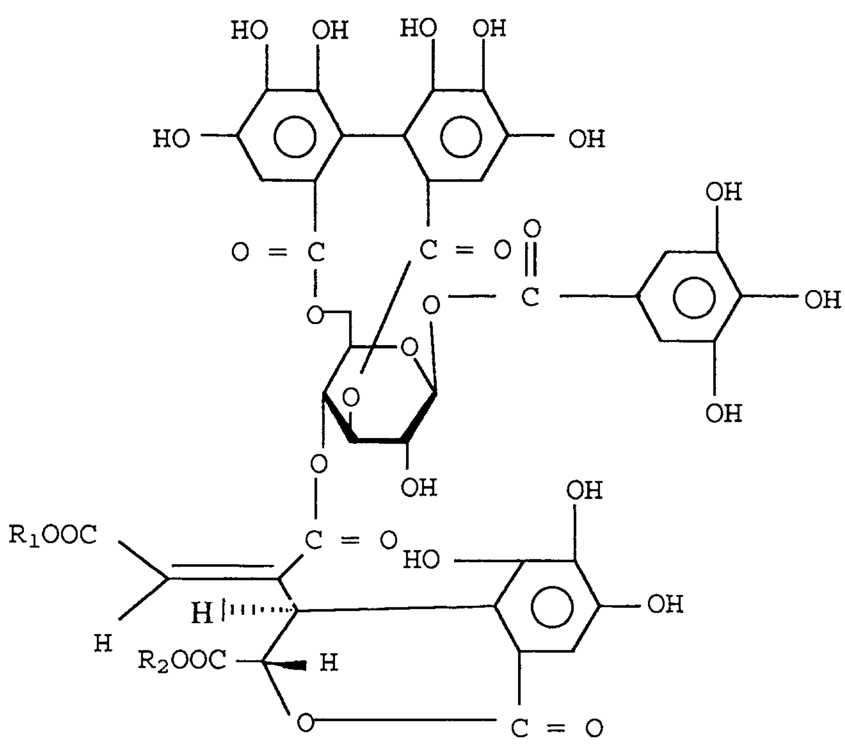

(wherein, $R_1$ and $R_2$ are the same or different and each independently represents hydrogen, sodium or potassium) (to be referred to as the "formula compounds"), and its pharmaceutically acceptable salt.

The formula compounds were heretofore completely unknown to possess anti-retroviral effects.

BEST MODE FOR CARRYING OUT THE INVENTION

The extract of the formula compounds, which is obtained by extracting Phyllanthus Niruri L., a plant native to Taiwan and other countries, with water, can be purified using commonly used column chromatography techniques. Examples of carriers used in the above-mentioned column chromatography include Sephadex LH-20, TSK gel 120T and cellulose. Examples of extracting solvents used include methanol, ethanol, water, acetic acid, acetonitrile, aqueous sodium hydrogenphosphate, benzene and hexane, etc., used alone or in the form of a mixed solvent. The number of times and the order in which column chromatography is run can be combined as is convenient.

Moreover, in the case a metallic salt is formed in the purification process, said metallic salt should be treated with hydrochloric acid and so forth before running the above-mentioned chromatography.

The following indicates specific examples of manufacturing the formula compounds.

Specific Example 1

42.7 g of dried Phyllanthus Niruri L. were extracted three times for 2 hours at 60°C using 400 ml of water, followed by distilling off the water from the extract to obtain 10.8 g of dry extract.

After dissolving this dry extract in chloroform, dissolving the chloroform insoluble portion in methanol, and further dissolving the methanol insoluble portion in water, the resulting soluble portion was dried to obtain 6.4 g of residue. This residue was then subjected to column chromatography (33ϕ x 260 mm) using Sephadex LH-20. After eluting with water, 181-360 ml of eluate was dried to obtain 230 mg of residue. This was then further subjected to column chromatography (20ϕ x 360 mm) using cellulose. After eluting with 2% aqueous acetic acid, 170 mg of a crude fraction was obtained from 81-140 ml of eluate. This crude fraction was subjected to column chromatography (22ϕ x 250 mm) using TSK gel 120T. After eluting (20 ml/min) with a mixture in which the concentration of acetonitrile was sequentially increased to 5-20% with respect to 10 mM aqueous sodium hydrogenphosphate, 280-360 ml of the eluted (20 ml/min.) fraction were further subjected to column chromatography (20ϕ x 500 mm) using Sephadex LH-20. After eluting with water, 191-250 ml of eluate were dried to obtain 28.4 mg of powder. The physicochemical properties of this powder were consistent with the physicochemical properties of repandusinic acid A monopotassium salt) described in the references (Chem. Pharm. Bull., 37(10), 2624-2630 (1989)).

Specific Example 2

After adding 1.5 ml of 1 N hydrochloric acid to 20 mg of the repandusinic acid A monosodium salt obtained in Specific Example 1 and allowing same to react, the solution was subjected to column chromatography (10ϕ x 100 mm) using Sephadex LH-20. The methanol soluble portion (4-7 ml) was then dried to obtain 15 mg of powder. The physicochemical properties of this powder were consistent with the physicochemical properties of repandusinic acid A described in the references (Chem. Pharm. Bull., 37-(10), 2624-2630 (1989)).

The following provides an explanation of experimental examples pertaining to the formula compounds having anti-retroviral effects.

Experimental Example 1

⟨Effects on Reverse Transcriptase Activity⟩

A reaction mixture having the composition indicated below was prepared.

| | |
|---|---|
| * Human immunodeficiency virus reverse transcriptase | 1 unit*/ml |
| * Template-primer complex in the form of polyadenylate-oligothymidylate complex (Polyadenylate polyrA, oligothymidylate $pdT_{12-18}$ (1:1), Pharmacia) | 5 μg/ml |
| * Tris-hydrochloric acid (pH 8.0) | 25 mM |
| * Potassium chloride | 75 mM |
| * Magnesium chloride | 8 mM |
| * Dithiothreitol | 2 mM |
| * [$^3$H]deoxythymidine triphosphate (1.55 TBq/mmol) | 0.2 μM |
| * Deoxythymidine triphosphate | 9.8 μM |
| * Compound of Specific Example 1 or Compound of Specific Example 2 | 0.05-1 μg/ml |
| * Purified water | Suitable amount |

* 1 unit refers to the specific activity unit for which reverse transcriptase consumes 1 nmol of dNTP (deoxynucleic acid triphosphate) in 10 minutes at 37°C.

50 μl of this reaction mixture was allowed to react for 30 minutes at 37°C.

Quantitative determination of the reaction product in the form of DNA was performed by measuring the uptake of the radioactivity of [$^3$H]deoxythymidine triphosphate into DNA using ion exchange filter paper, taking that to represent reverse transcriptase inhibitory activity, and calculating the 50% inhibitory concentration ($IC_{50}$) of the test compound. As a result, the $IC_{50}$ values of both the compounds of Specific Example 1 and Specific Example 2 were 0.1 μg/ml, confirming that the compounds possess anti-retroviral effects based on inhibitory action of reverse transcriptase.

Experimental Example 2

⟨Test of Anti-AIDS Virus Activity Using Clone H9 Cells (T-Cells)⟩

1 x $10^6$ clone H9 cells (©T-cells) were placed in individual culture tubes. The serially diluted test drug was then placed in each tube. Roughly 100$TCID_{50}$ of human immunodeficiency virus (culture supernatant of H9/HTLV-$III_B$ cells) were then placed in each tube to infect the above-mentioned cells. After incubating the tubes for 1 hour at 37°C in the presence of 5% $CO_2$, the unabsorbed viral particles and test substance were removed by centrifugal separation (x3000 g, 5 minutes). Moreover, after again centrifugally washing (x3000 g, 5 minutes) the cells with fresh culture liquid, fresh culture liquid was added to each tube to prepare cell suspensions having a concentration of 0.2 x $10^6$ cells per milliliter. 0.5 ml of these cell suspensions were added to each well of a 24 well culture plate containing 0.5 ml of the serially diluted test drug. After incubating this plate for 5 days at 37°C in the presence of 5% $CO_2$, the amount of p24, which is the human immunodeficiency virus antigen in the culture supernatant, was measured, followed by calculation of the rate of inhibition (%) at the concentrations of the test drug indicated below. Those results are indicated in Table 1.

Table 1

| | | Rate of Inhibition (%) |
|---|---|---|
| Compound of Specific Example 1 | 0.195 μg/ml<br>3.125 μg/ml<br>12.5 μg/ml | 27.8%<br>66.7%<br>94.4% |

The anti-retroviral agent of the present invention demonstrated excellent anti-HIV virus action, as well as a low level of cellular toxicity, causing no damage to the test cells even at a concentration of 20 μg/ml.

Moreover, since the formula compound suppresses retrovirus growth by inhibiting reverse transcriptase activity, which is required for the growth of a retrovirus, it can be applied to any type of virus so long as said virus is a retrovirus.

Specific examples of retroviruses include leukemia virus, sarcoma virus, breast carcinoma virus, visna virus, maedi virus, HIV and HTLV-I.

In addition, the formula compounds were confirmed to have a high degree of safety as there were no cases of death following oral administration (100 mg/kg) to mice.

The following provides an explanation of the dosage and preparation of the formula compounds.

The formula compounds can be administered to animals and humans as is or together with a commonly used preparation carrier. There are no particular limitations on the administration form, and can be suitably selected and used as necessary. Examples of administration forms include oral preparations such as tablets, capsules, granules, grains and powders, and non-oral preparations such as injection preparations and suppositories.

In order to demonstrate the expected effects in the form of an oral preparation, a normal adult dosage of 0.1-6 g, in terms of the weight of the formula compounds, divided among several administrations per day is considered to be suitable, although these vary according to age, weight and severity of the disorder of the patient.

In the present invention, oral preparations, such as tablets, capsules or granules, can be manufactured according to routine methods using, for example, starch, lactose, saccharose, mannitol, carboxymethyl cellulose, corn starch or inorganic salts.

In addition to the above-mentioned suitable vehicles, binders, disintegrating agents, surface activators, glossing agents, fluidity promoters, flavoring agents, coloring agents and perfumes, etc., can be used in this type of preparation. Respective specific examples are as indicated below.

[Binders]

Starch, dextrin, Arabic gum, gelatin, hydroxypropyl starch, methyl cellulose, sodium carboxymethyl cellulose, hydroxypropyl cellulose, crystal cellulose, ethyl cellulose, polyvinyl pyrrolidone and macrogall.

[Disintegrating Agents]

Starch, hydroxypropyl starch, sodium carboxymethyl cellulose, calcium carboxymethyl cellulose, carboxymethyl cellulose and lower substituted hydroxypropyl cellulose.

[Surface Activators]

Sodium lauryl sulfate, soy bean lecithin, sucrose fatty acid ester and polysorbate 80.

[Glossing Agents]

Talc, waxes, hydrogenated vegetable oil, sucrose fatty acid ester, magnesium stearate, calcium stearate, aluminum stearate and polyethylene glycol.

[Fluidity Promoters]

Light silicic anhydride, dry aluminum hydroxide gel, synthetic aluminum silicate and magnesium silicate.

In addition, the formula compound can also be administered in the form of a suspension, emulsion, syrup or elixir, and these various preparation forms may contain flavoring agents, aromatic agents and

coloring agents.

In order to demonstrate the expected effects in the form of a non-oral preparation, a normal adult dosage of 1-100 mg per day, in terms of the weight of the formula compound, administered either by intravenous injection, intravenous drip, subcutaneous injection or intramuscular injection is considered to be suitable, although these vary according to age, weight and severity of the disorder of the patient.

This non-oral preparation can be manufactured according to routine methods. Typical examples of diluents that can be used include distilled water for intravenous injection, physiological saline, aqueous glucose solution, vegetable oil for intravenous injection, sesame oil, peanut oil, soy bean oil, corn oil, propylene glycol and polyethylene glycol. Moreover, disinfectants, preservatives or stabilizers may also be added as necessary. In addition, with respect to stability, this oral-preparation can be frozen after charging vials or other suitable containers. The liquid can then be re-prepared from the freeze-dried product immediately before use by removing moisture using routine freeze-drying technology. Moreover, suitable isotonic agents, stabilizers, preservatives, analgesics and so forth may also be added as necessary.

Examples of other non-oral preparations include applied preparations such as external liquids and ointments, and suppositories and so forth for intrarectal administration, which can also be manufactured according to routine methods.

Embodiment 1

| | |
|---|---|
| (1) Crystal cellulose | 84.5 g |
| (2) Magnesium stearate | 0.5 g |
| (3) Calcium carboxymethyl cellulose | 5 g |
| (4) Compound of Specific Example 1 | 10 g |
| Total | 100 g |

Ingredients (1), (4) and a portion of (2) are uniformly mixed following the above-mentioned prescription. After compression molding the mixture, the mixture is crushed followed by the addition of ingredient (3) and the remainder of ingredient (2), and mixing. The mixture is then compression molded in a tablet making machine to obtain 200 mg tablets.

Each of these tablets contains 20 mg of the compound of Specific Example 1, 5-7 of these tablets are administered to a normal adult per day over the course of several administrations.

Embodiment 2

| | |
|---|---|
| (1) Crystal cellulose | 34.5 g |
| (2) 10% hydroxypropyl cellulose ethanol solution | 50 g |
| (3) Calcium carboxymethyl cellulose | 5 g |
| (4) Magnesium stearate | 0.5 g |
| (5) Compound of Specific Example 2 | 10 g |
| Total | 100 g |

Ingredients (1), (2) and (5) are uniformly mixed following the above-mentioned prescription. After kneading the mixture according to routine methods, the mixture is formed into granules by an extrusion granulating machine. Following drying and crushing, ingredients (3) and (4) are mixed followed by compression molding in a tablet making machine to obtain 200 mg tablets.

Each of these tablets contains 20 mg of the compound of Specific Example 2, 5-7 of these tablets are administered to a normal adult per day over the course of several administrations.

Embodiment 3

| | |
|---|---|
| (1) Distilled water for injection<br>(2) Glucose<br>(3) Compound of Specific Example 1 | Suitable amt.<br>200 mg<br>10 mg |
| Total | 15 ml |

After dissolving ingredient (2) and ingredient (3) in the distilled water for injection, the solution is filled into 5 ml ampules to obtain an injection preparation following pressure sterilization for 15 minutes at 121 °C.

INDUSTRIAL APPLICABILITY

The present invention is effective in the treatment of viral diseases caused by retrovirus.

**Claims**

1. An anti-retroviral agent comprising, as an active ingredient, a compound represented by the following formula:

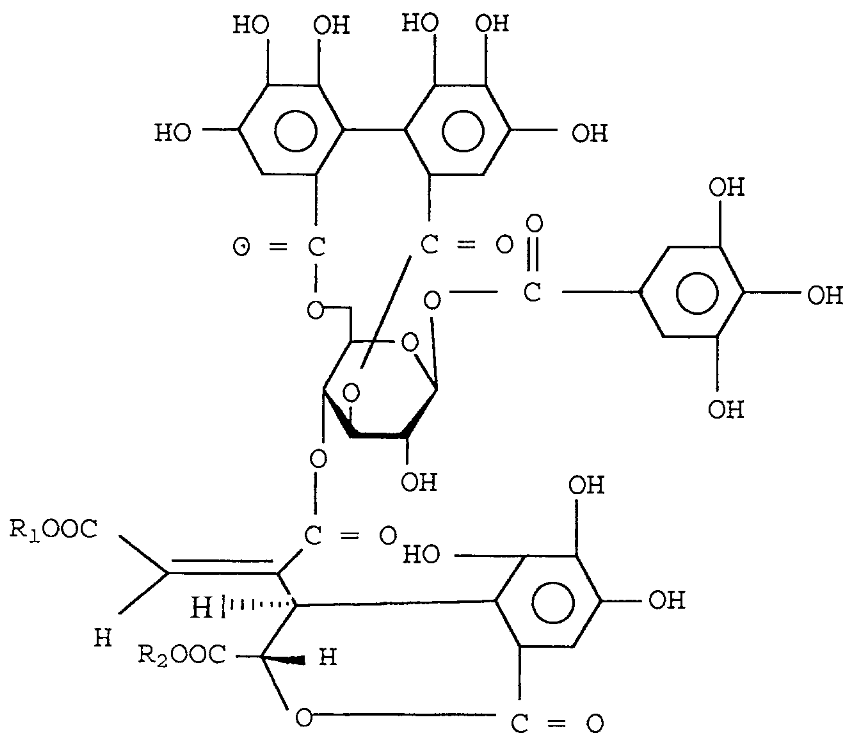

(wherein, $R_1$ and $R_2$ are the same or different and each independently represent hydrogen, sodium or potassium), or its pharmaceutically acceptable salt.

2. A method for the treatment of viral diseases caused by a retrovirus, comprising the administration of an effective amount of the anti-retroviral agent according to claim 1.

# INTERNATIONAL SEARCH REPORT

International Application No PCT/JP91/00651

**I. CLASSIFICATION OF SUBJECT MATTER** (if several classification symbols apply, indicate all) [6]

According to International Patent Classification (IPC) or to both National Classification and IPC

Int. $Cl^5$ A61K31/70//C07H13/08

**II. FIELDS SEARCHED**

Minimum Documentation Searched [7]

| Classification System | Classification Symbols |
|---|---|
| IPC | C07H13/00-17/04, A61K31/70, A61K35/78 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 57-91922 (Yasuo Tanaka), June 8, 1982 (08. 06. 82) | 1 |
| A | Chemical & Pharmaceutical Bulletin, Vol.37, No.10, pages 2624 to 2630 (1989) | 1 |
| A | Chemical & Pharmaceutical Bulletin, Vol.38, No.8, pages 2162 to 2171 (1990) | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| July 17, 1991 (17. 07. 91) | August 5, 1991 (05. 08. 91) |
| International Searching Authority | Signature of Authorized Officer |
| Japanese Patent Office | |